# EUROPEAN PATENT APPLICATION

(11) **EP 1 340 462 A1**
(43) Date of publication of application: **03.09.2003**
(21) Application number: 03251141.2
(22) Date of filing: 26.02.2003
(51) Int. Cl.: A61B 17/11

(54) **Sponge for creating an anastomosis between vessels**

(30) Priority: 28.02.2002 US 85738
(71) Applicant: ETHICON, INC., Somerville New Jersey 08876 (US)
(72) Inventor: Weadock, Kevin S., Princeton, NJ 08540 (US); Kizer, Beth Ann, Columbus, Ohio 43212 (US); Pancake, Mary Hoffman, Gahanna, Ohio 43230 (US); Gleeson, James B., Columbus, Ohio 43212 (US)
(74) Representative: Merrifield, Sarah Elizabeth

(57) **Abstract**

A method for creating an anastomosis between first and second vessels. The method includes: attaching a portion of the first vessel to a body, where the body is at least partly fabricated from a sponge material; attaching a portion of the second vessel to the body; and creating an anastomosis between the portions of the first and second vessels and through an opening in the body. The attaching of the first vessel to the body includes either attaching an end portion of the first vessel to the opening formed in the body or attaching a side portion of the first vessel to the body. The attaching of the second vessel to the body includes attaching a side portion of the second vessel to the body.

## Description

### BACKGROUND OF THE INVENTION

### 1. Field of the Invention

The present invention relates generally to creating an anastomosis between vessels, and more particularly, to a sponge for creating an anastomosis between vessels.

### 2. Prior Art

Saphenous veins, internal mammary arteries (IMA), and radial arteries are used as conduits in coronary artery bypass surgery (CABG). Conventional techniques for harvesting these vessels are known in the art. After harvesting a suitable graft vessel, the heart is accessed, either through conventional open heart surgery or endoscopically. An anastomosis necessary to bypass the lesions in the coronary arteries is then performed. In conventional open heart surgery, the surgeon sutures the proximal end of each graft vessel to the patient's aorta and the distal end to the diseased coronary artery, distal to the blockage or lesion. Recently, performing the CABG procedure endoscopically is becoming more popular due to the reduced trauma to the patient and its associated benefits. However, anastomosis of the vessels during a CABG procedure by suturing is very difficult and time consuming, particularly in beating heart or "off-pump" procedures. Furthermore, other known techniques for anastomosis do not lend themselves to being performed endoscopically due to the size and shape constraints inherent in endoscopic instruments.

Additionally, the CABG procedure can be a beating heart CABG in which the patient's heart continues to beat during the procedure, or a stopped heart procedure in which the heart is temporarily stopped and a cardiopulmonary bypass machine (CPB) is used to provide the function of the heart during the procedure. During the beating heart CABG, the coronary artery is ligated to stop blood flow therein while the necessary anastomosis is performed. With conventional suturing in beating heart CABG, the coronary artery is ligated for as much as 10 minutes while the vessels are anastomosed.

Lastly, automated mechanical devices known in the art for creating an anastomosis between vessels tend to be rigid structures which may result in a compliance mismatch between the vessels and the device, potentially causing intimal hyperplasia and subsequent thrombosis. The mechanical devices of the prior art also tend to cause trauma to the vessels themselves due to loading and eversion of the vessels.

### SUMMARY OF THE INVENTION

Therefore it is an object of the present invention to provide device and methods for creating an anastomosis between vessels, which can be performed easily in an endoscopic or open procedure and which avoids vessel trauma like loading, eversion, and the like.

It is another object of the present invention to provide device and methods for creating an anastomosis between vessels, which eliminates the need for suturing.

It is yet another object of the present invention to provide device and methods for creating an anastomosis between vessels, which can be done relatively quickly as compared to the methods of the prior art.

It is yet another object of the present invention to provide device and methods for creating an anastomosis between vessels, which minimizes the time the coronary artery is ligated in a CABG procedure.

It is still yet another object of the present invention to provide device and methods for creating an anastomosis between vessels, which provides a compliant coupling for reducing the likelihood of intimal hyperplasia at the anastomotic site.

Accordingly, a device for creating an end-to-side anastomosis between first and second vessels is provided. The device comprises: a body, at least a portion of which is fabricated from a sponge material, the body having an opening for insertion of an end of the first vessel therein; first securing means for securing the first vessel in the opening; and second securing means for securing a side of the second vessel to the body such that a hole formed in the side of the second vessel is in fluid communication with the end of the first vessel.

The first securing means preferably comprises an adhesive disposed between an outer surface of the first vessel and a corresponding surface of the hole. The first securing means further preferably comprises sealing means for sealing the outer surface of the first vessel against the corresponding surface of the opening. The sealing means preferably comprises a catheter having a balloon, the catheter being disposed in the first vessel such that when inflated, the balloon urges the outer surface of the first vessel against the corresponding surface of the opening to sandwich the adhesive therebetween.

The second securing means preferably comprises an adhesive disposed between an outer surface of the second vessel and a corresponding surface of the body. The adhesive is preferably disposed on at least a first vessel-contacting surface of the opening. Where the sponge material contains pores, the adhesive is further preferably disposed in the pores of the sponge material.

The device preferably further comprises an alignment means for aligning the opening with the hole in the second vessel. The alignment means preferably comprises a radially compressible member having a cylindrical body embedded in the body circumferentially about the opening, the radially compressible member further having a plurality of pins protruding from the body. At least a portion of the radially compressible member is preferably fabricated from a resorbable material as is the sponge material.

Where the sponge material contains pores, the device preferably further comprises a medicating agent disposed in at least a portion of the pores of the sponge material. The medicating agent is preferably an anastomosis modulating agent.

Also provided is a device for creating a side-to-side anastomosis between first and second vessels. The device comprises: a body, at least a portion of which is fabricated from a sponge material, the body having an opening formed therein; first securing means for securing a side of the first vessel to the body; and second securing means for securing a side of the second vessel to the body such that a hole formed in the side of the first and second vessels are each in fluid communication with the opening.

The body is preferably disk-shaped and has a slot of semicircular cross-section corresponding to each of the first and second vessels for acceptance thereof, where each of the slots communicate through the opening.

Preferably, at least one of the first and second securing means comprises an adhesive disposed between an outer surface of the first and/or second vessel and a corresponding surface of the body. The at least one of the first and second securing means preferably further comprises sealing means for sealing the outer surface of the first and/or second vessel against the corresponding surface of the body. The sealing means preferably comprises a catheter having a balloon, the catheter being disposed in the first and/or second vessel such that when inflated, the balloon urges the outer surface of the first and/or second vessel against the corresponding surface of the body to sandwich the adhesive therebetween. The adhesive is preferably disposed on at least a vessel-contacting surface of the body. Where the sponge contains pores, the adhesive is preferably disposed in pores of the sponge material.

Preferably, the device further comprises an alignment means for aligning the opening with at least one of the holes in the first and second vessels. The alignment means preferably comprises a radially compressible member having a cylindrical body embedded in the body circumferentially about the opening, the radially compressible member further having a plurality of pins protruding from the body. Preferably, at least a portion of the radially compressible member is fabricated from a resorbable material, as is the sponge material.

Where the sponge material contains pores, the device preferably further comprises a medicating agent disposed in at least a portion of the pores of the sponge material. The medicating agent is preferably an anastomosis modulating agent.

Still yet provided is a method for creating an anastomosis between first and second vessels. The method comprises: attaching a portion of the first vessel to a body, the body being at least partly fabricated from a sponge material; attaching a portion of the second vessel to the body; and creating an anastomosis between the portions of the first and second vessels and through an opening in the body.

In a first version, the attaching of the portion of the first vessel to the body comprises attaching an end portion of the first vessel to the opening formed in the body and the attaching of a portion of the second vessel to the body comprises attaching a side portion of the second vessel to the body. In which case, the creating of the anastomosis between the portions of the first and second vessels preferably comprises forming a hole in the portion of the second vessel corresponding to the end of the first vessel and the opening in the body. The forming of the hole can be subsequent to the attaching of the portion of the second vessel to the body. Alternatively, the forming of the hole can be prior to the attaching of the portion of the second vessel to the body.

In a second version, the attaching of the portion of the first vessel to the body comprises attaching a side portion of the first vessel to the body and the attaching of the portion of the second vessel to the body comprises attaching a side portion of the second vessel to the body. In which case, the creating of the anastomosis between the portions of the first and second vessels preferably comprises forming holes in the side portions of the first and second vessels corresponding to each other and the opening in the body. The forming of at least one of the holes in the side portions of the first and second vessels can be subsequent to the attaching of the corresponding side portion to the body. Alternatively, the forming of at least one of the holes in the side portions of the first and second vessels can be prior to the attaching of the corresponding side portion to the body.

At least one of the attaching of the portion of the first and second vessels to the body preferably comprises adhering the portion to the body with an adhesive disposed between a corresponding outer surface of the vessel and the body. The method preferably further comprises sealing the corresponding outer surface of the vessel to the body. Preferably, the sealing comprises inflating a balloon in a lumen of the vessel to urge the corresponding outer surface against the body and to sandwich the adhesive therebetween.

The method preferably further comprises aligning a hole in one of the first and second vessels with the opening in the body.

### BRIEF DESCRIPTION OF THE DRAWINGS

These and other features, aspects, and advantages of the apparatus and methods of the present invention will become better understood with regard to the following description, appended claims, and accompanying drawings where:
Figure 1A illustrates an isometric view of a first version of a preferred implementation of the device for creating an end-to-side anastomosis between first and second vessels.
Figure 1B illustrates an isometric view of the device of Figure 1A having an end of a first vessel attached thereto.
Figure 1C illustrates an isometric view of the device of Figure 1B having a side of a second vessel attached thereto.
Figure 2A illustrates a sectional view of the device of Figure 1C.
Figure 2B illustrates the sectional view of Figure 2A having a sealing means in the first vessel.
Figure 3A illustrates an isometric view of a second version of a preferred implementation of the device for creating an end-to-side anastomosis between first and second vessels.
Figure 3B illustrates an isometric view of the device of Figure 3A having an end of a first vessel attached thereto.
Figure 3C illustrates an isometric view of the device of Figure 3B having a side of a second vessel attached thereto.
Figure 4 illustrates a sectional view of the device of Figure 3C.
Figure 5A illustrates an isometric view of a preferred implementation of a device for creating a side-to-side anastomosis between first and second vessels.
Figure 5B illustrates an isometric view of the device of Figure 5A having sides of the first and second vessels attached thereto.
Figure 5C illustrates a sectional view of the device of Figure 5B showing a catheter and cutting means in the lumen of the second vessel.

### DETAILED DESCRIPTION OF THE PREFERRED EMBODIMENT

Although this invention is applicable to numerous and various types of vessels to be anastomized, it has been found particularly useful in the environment of blood vessels and further particularly useful in anastomizing a harvested graft vessel to a coronary vessel in a CABG procedure. Therefore, without limiting the applicability of the invention to blood vessels or anastomizing blood vessels in a CABG procedure, the invention will be described in such environment.

In general, the present invention relates to devices and methods for creating an anastomosis between a first and second vessel with a body, at least a portion of which is fabricated from a flexible material. Although the devices and methods of the present invention are described by way of example with regard to blood vessels, the term "vessel" is used generally to mean any tubular body structure, such as bowels, veins, peripheral arteries, and ducts. Furthermore, the terms "sponge" or "sponge material" are used generally to mean any flexible elastomer, polymer or natural material having a porous and/or cellular structure as well as a non-porous and non-cellular structure.

Two preferred versions of the device will now be described by way of example only and not to limit the spirit or scope of the present invention. The first version of the device creates an end-to-side anastomosis between an end of a first vessel and the side of a second vessel while the second version of the device creates a side-to-side anastomosis between a side of the first vessel and a side of the second vessel.

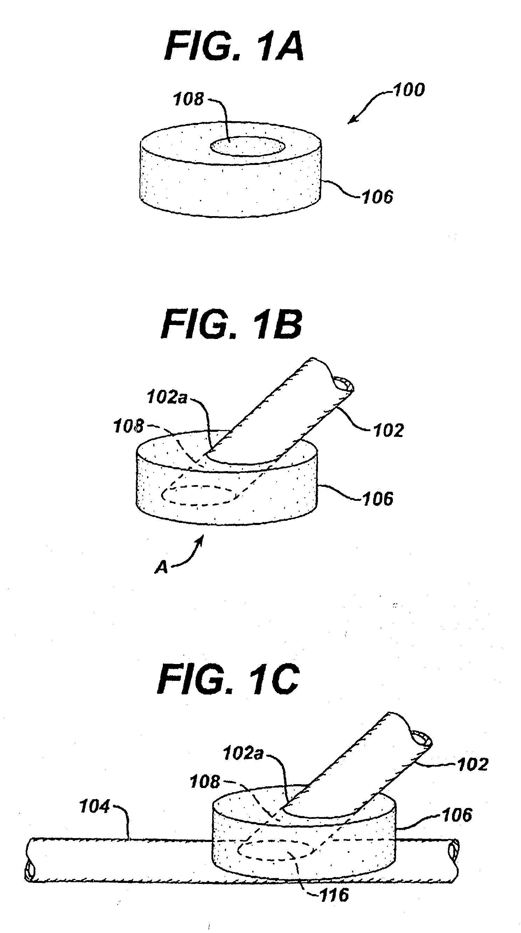

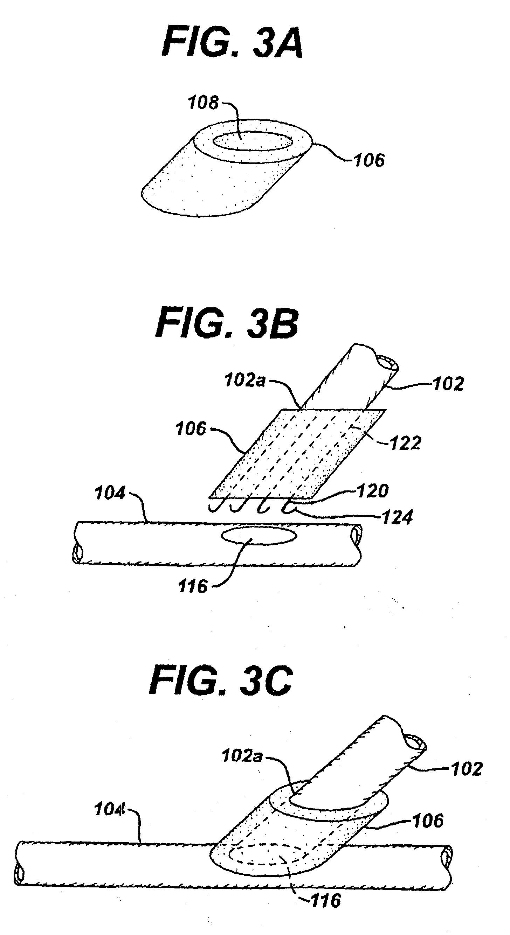

Referring now to Figures 1A, 1B, and 1C, a first version of the device for creating an anastomosis between first and second vessels 102, 104 is shown, the device being generally referred to by reference numeral 100. Specifically, the first version of the device shown in Figures 1A, 1B, and 1C is particularly configured for creating an end-to-side anastomosis between the first and second vessels 102, 104. The device comprises a body 106. At least a portion of the body 106, and preferably the entire body 106 is fabricated from a sponge material, such as collagen, gelatin, poly-glactic acid (PGA) and polylactic acid (PLA) copolymers, hydrogels, oxidized regenerated cellulose (ORC), expanded Teflon, silicone, and poly-vinyl alcohol (PVA). As discussed above, the sponge material can be porous, open cell, closed cell, non-porous, or non-cellular. The sponge material can be of a type that does not break down over time or resorbable, which does break down over time. Such resorbable materials are well known in the medical arts. Those skilled in the art will appreciate that the body 106 can be formed in any number of shapes and sizes, such as a disk-shape shown in Figure 1A or a sleeve-shape as shown in Figures 3A, 3B and 3C.

Referring back to Figure 1A, the body 106 has an opening 108 for insertion of an end 102a of the first vessel 102 therein. The opening 108 can be any shape or size in cross-section to accommodate the end 102a of the first vessel 102, such as circular, elliptical, or oval. Furthermore, the opening 108 can be parallel with a central axis of the body 106 or offset therefrom. The opening 108 is preferably a through hole that pierces completely through the body 106. Figure 1B shows the end 102a of the first vessel 102 inserted into the opening 108.

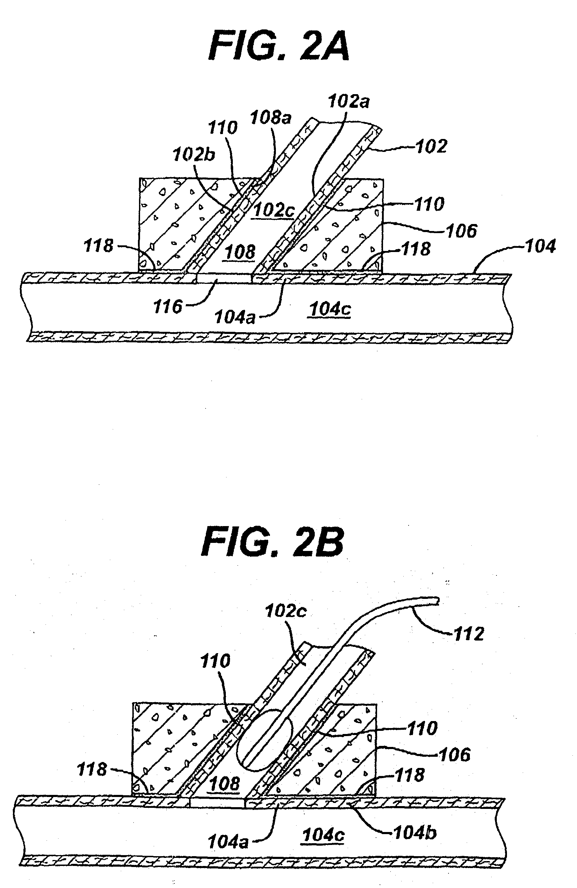

Referring now to Figures 2A and 2B, the device includes a first securing means for securing the end 102a of the first vessel 102 in the opening 108. The first securing means preferably comprises an adhesive 110 disposed between an outer surface 102b of the first vessel 102 and a corresponding surface 108a of the opening 108. The adhesive 110 is preferably disposed on the surface of the opening 108 and/or in the pores or cells of the sponge material of the body 108, if applicable. Internal medical adhesives for use in the sponge device of the present invention are well known in the art and include cyanoacrylates, fibrin glues, and collagen glues and sealants. Preferably, the adhesive is not placed in the blood path of the vessels.

In addition to the adhesive 110, a medicating agent, such as an anastomosis modulating agent, can also be disposed on the surface of the body 106 or in at least a portion of the pores (or cells) of the sponge material of the body 106. The anastomizing modulating agent can modulate or regulate the healing response at the anastomotic site. Examples of anastomosis modulating agents include anti-proliferatives such as sirolimus, papaverine, antibiotics, angiogenic factors (VEGF), anti-inflamatory agents, and anticoagulants such as heparin.

The first securing means preferably further comprises sealing means for sealing the outer surface 102b of the first vessel 102 against the corresponding surface 108a of the opening 108. The sealing means preferably comprises a catheter 112 having an inflatable balloon 114 at an end thereof. The catheter 112 is disposed in a lumen 102c of the first vessel 102 such that when inflated, the balloon 114 urges the outer surface 102b of the first vessel 102 against the corresponding surface 108a of the opening 108 to sandwich the adhesive 110 therebetween. In addition, the catheter 112 and inflatable balloon 114 can be introduced from direction A shown in Figure 1B prior to attachment of the second vessel 104 to the body 106.

The sealing of the end 102a of the first vessel 102 to the surface 108a of the opening 108 can be done before or after the sponge is attached to the second vessel 104. The use and construction of such balloon catheters are well known in the medical arts. The use of a balloon catheter as the sealing means is given by way of example only, other such means can be utilized, such as a flexible shaft (not shown) having a blunt tapered tip, which is introduced into the lumen 102c of the first vessel 102 in direction A to urge the outer surface 102b of the first vessel 102 against the surface 108a of the opening 108.

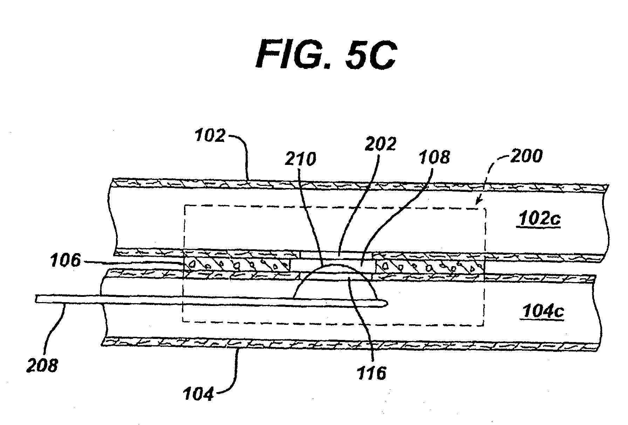

The device also includes a second securing means for securing a side 104a of the second vessel 104 to the body 106 such that a hole 116 formed in the side 104a of the second vessel 104 is in fluid communication with the end 102a (and thus, the lumen 102c) of the first vessel 102. The hole 116 can be formed in the second vessel 104 either before or after the second vessel 104 is attached to the body 106 by conventional techniques known in the art. If made before attachment to the body 106, the hole 116 is preferably formed by cutting a slit in the wall of the second vessel 104, which when pressurized by a fluid therein, such as blood, opens to an elliptical shape. If the hole 116 is formed after attachment of the second vessel 104 to the body 106, the hole 116 is preferably formed by passing a catheter into a lumen 104c of the second vessel 104 where the catheter has a cutting means for forming the hole 116. Such a catheter and cutting means is described below with regard to Figure 5C.

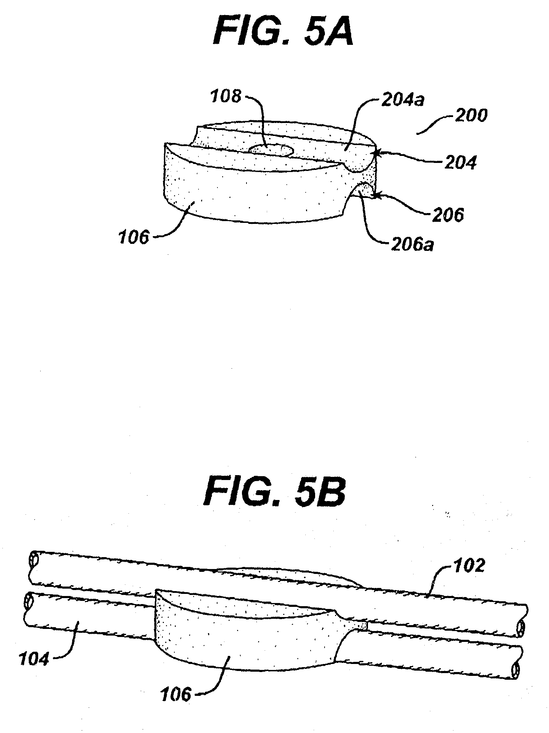

The second securing means preferably comprises an adhesive 118 disposed between an outer surface 104b of the side 104a of the second vessel 104 and a corresponding surface 108 108b of the body 108. As discussed above, the adhesive 118 is preferably disposed on the surface of the body 106 which corresponds to the portion of the second vessel 104 to be adhered and can also be disposed in the pores or cells of the sponge material of the body 108, if applicable. Figure 1C shows the first and second vessels 102, 104 attached to the body 106. As will be discussed below with regard to Figure 5A, the body 106 can be shaped to position and accommodate the second vessel, for instance in a slot of semi-circular cross-section.

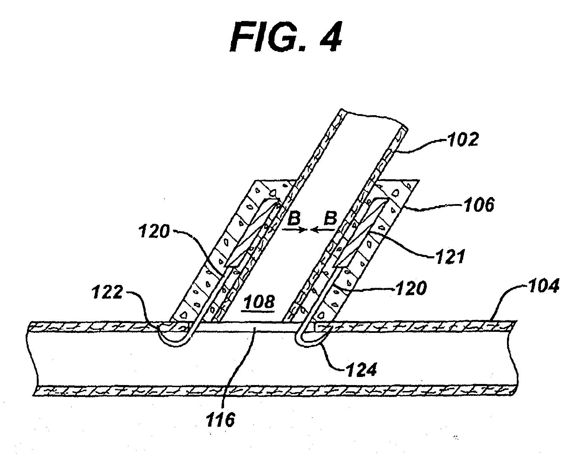

Referring now to Figures 4, also preferably provided is an alignment means, which preferably comprises a member having a plurality of pins 120 surrounding a cylindrical body 121 (shown in cross-section), which is compressible in the radial direction. The use of such radially compressible members are known in the art, such as in U.S. Patent No. 5,695,504 to Gifford, the disclosure of which is incorporated herein in its entirety by its reference.

Preferably, the cylindrical body 121 of the mechanical device is embedded in the body 106 such that an end 124 of the pins 120 protrude from the body 106 adjacent the opening 108. Preferably, the ends 124 encircle the opening 108. The tips 122 of the ends 124 can by pointed or blunt. The pins 120 and cylindrical body 121 can be fabricated from any medically approved material for internal use, such as nitinol, stainless steel, tantalum, titanium, and nickel alloys. The pins 120 may also be fabricated from a resorbable material, such as PLA, PGA, or PLA-PGA copolymers, which breaks down over time after the need for alignment is no longer necessary.

To secure the second vessel 104 to the body 106, the adhesive 118 is loaded on the appropriate surface of the body 106 or it is exposed by removing a peel-away strip. The cylindrical body 121 of the mechanical anastomotic device is compressed radially in direction B to collapse the same along with the body 106 and first vessel 102 adhered thereto. The compression is preferably performed with a grasping tool such as a forceps which is a standard size or which is customized to fit the body 106 within its jaws. The pin ends 124 are placed in the hole 116 in the second vessel 104 and the compression of the cylindrical body 121 is released. After release of the radial compression, the cylindrical body 121 expands resulting in the pins 120 engaging the side portions of the hole 116 to align the opening 108 in the body 106 with the hole 116 in the vessel. The tips 122 of the pins 124 engage at least a part of the second vessel's 104 wall. The tips 124 can either pierce the sidewall of the second vessel 104 to aid in securing the second vessel 104 to the body 108 or merely urge against the sidewall of the second vessel 104. The alignment means described above is given by way of example only and not to limit the scope or spirit of the present invention. Those skilled in the art will appreciate that other alignment means are possible, for instance, in an endo-vascular approach to creating an anastomosis with the devices of the present invention, a balloon catheter can be used for alignment purposes.

Referring now to Figures. 5A, 5B, and 5C, there is illustrated a device for creating a side-to-side anastomosis between first and second vessels 102, 104, the device being generally referred to by reference number 200 with like features being referred to by like reference numerals from the preceding Figures. The device 200 comprises a body 106, at least a portion of which is fabricated from a sponge material. However, as discussed above, it is preferred that the entire body 106 be fabricated of the sponge material. The body 106 has an opening 108 formed therein, similarly to that described above.

The first and second securing means are similar to that described above, however, instead of an end of the first vessel 102 being attached to the body 106, a side 102d of the first vessel 102 is attached to the body 106. The side 104a of the second vessel 104 is attached to the body *in* a similar manner as described above. The first and second vessels 102, 104 are secured to the body 106 such that a hole 116, 202 formed in the side of each of the first and second vessels 102, 104 are in fluid communication with the opening 108.

Preferably, the body 106 is disk-shaped and has a slot 204, 206 of semicircular cross-section corresponding to each of the first and second vessels 102, 104 for acceptance thereof. The slots 204, 206 communicate through the opening 108 and also position and orient the first and second vessels 102, 104 relative to the each other and the body 106. Although, the first and second vessels 102, 104 are shown oriented in a parallel in-line" configuration, they can be oriented with respect to each other by an angle without departing from the scope or spirit of the present invention. For instance, the slots 202, 204, and thus, the first and second vessels 102, 104 can be oriented 90 degrees with respect to each other.

As discussed above, the preferred means for securing the first and second vessels 102, 104 to the body 106 comprises adhering the same with a proper medical grade adhesive for internal use, which is preferably disposed on appropriate surfaces of the body 106 and/or in the pores or cells of the sponge material of the body 106, as can be a medicating agent, such as an anastomosis modulating agent. Furthermore, pins 120 can be utilized to align the opening 108 in the body 106 with the hole 116 in the second vessel 102. Similarly to that discussed above, a catheter having a balloon can be used to seal the respective surfaces 102d, 104a, of the first arid second vessels 102, 104 to corresponding surfaces 204a, 206a of the slots 204, 206.

Referring now to Figure 5C, there is illustrated, in cross-section, the device 200 of Figure 5A secured to first and second vessels 102, 104. As discussed briefly above, the holes 116, 202 in the first and/or second vessels can be formed either before or after its respective vessel is secured to the body 106. If formed prior to attachment to the body 106, the hole 116, 202 can be formed by simply making an incision (slit) in the wall of the vessel. In a CABG procedure, it is preferred that the graft vessel (e.g., 102) be prepared prior to being attached to the body 106 by forming the hole (e.g., 202) with an incision. However, since the other vessel (e.g., 104) may need to be ligated if its hole (e.g., 116) were made prior to securing the vessel to the body 106, it is preferred that the hole in such vessel be formed after attachment of the vessel to the body 106, thereby reducing or eliminating the need for ligation of the vessel. However, it will be appreciated by those skilled in the art that both holes 116, 202 can be formed after attachment of their respective vessels 102, 104 to the body 106, and that they can also be formed simultaneously.

The formation of at least one of the holes 116, 202 after attachment of their respective vessels 102, 104 to the body 106 is/are preferably formed by inserting a catheter 208 into one of the lumens 102c, 104c of the first and second vessels 102, 104 using known techniques for doing so. The catheter 208 has a cutting means at an end thereof, such as a retractable cutting wire 210 shown in Figure 5C. As is known in the art, the cutting wire 210 can be sharpened or electrosurgical.

### EXAMPLE

The use of the devices of the present invention will now be explained by way of an example and with reference to the Figures. As discussed above, the present invention has particular utility in a coronary artery bypass graft procedure (CABG). However, the use of the devices of the present invention are described with regard to the CABG procedure by way of example only and not to limit the scope or spirit of the present invention. A patient is prepared for cardiac surgery in a conventional manner using conventional techniques and procedures. The patient is then anesthetized and ventilated using conventional techniques. A conventional CABG procedure is performed by harvesting the greater saphenous vein from one or both of the patient's legs. Alternatively, other graft conduits such as the internal mammary artery or radial artery could be used. The surgeon prepares an access to the heart with conventional endoscopic instruments and techniques. The surgeon next begins dissecting the internal mammary artery (IMA) from the chest wall of the patient, so that the distal end of the vessel may be anastomosed to the diseased lower anterior descending (LAD) coronary artery on the distal side of a lesion on the septum near the left ventricle of the heart as a source of oxygenated blood. During the surgical procedure, the surgeon preferably elects to have the patient's heart beating to perform a conventional beating heart CABG, although the surgeon has a cardiopulmonary bypass machine (CPB) primed with the patient's blood and available if it is necessary to convert the beating heart procedure into a conventional stopped heart procedure.

The surgeon prepares the heart for attaching the graft vessels by cutting and pulling away the pericardium. After checking the graft vessels for patency, collateral damage and viability, the surgeon prepares to do the anastomoses necessary to bypass the lesions in the coronary arteries. The surgeon anastomoses the proximal end of each graft vessel to the patient's aorta and the distal end to the diseased coronary artery, distal to the blockage or lesion and anastomoses the distal end of the LAD to a coronary artery distal to a lesion with the devices and methods of the present invention.

In an end-to-side anastomosis, the surgeon or assistant then preloads the adhesive 110 into the opening 108 and inserts the distal end of the graft (first) vessel 102 into the opening 108 with the very distal end passing through and overhanging slightly through the opening 108. The catheter 112 with balloon 114 is inserted into the lumen 102c of the graft vessel 102 in the direction of arrow A. The balloon 114 is inflated to seal the outer surface 102b of the graft vessel 102 to the surface 108a of the opening 108. The overhanging portion of the graft vessel is then trimmed flush with the lower surface of the body 106. The target (second) vessel 104 is then ligated to create a bloodless field and a hole 116 is formed in the target vessel using instruments and methods known in the art. The adhesive 118 on the lower surface of the body 108 is then applied or exposed and the cylindrical body 121 of the mechanical anastomotic device and the body 106 are radially compressed in the direction of arrow B. The pin ends 124 are placed in the hole 116 and the radial compression is released allowing the pins 120 to engage the side surfaces of the hole 116 and align the hole 116 with the opening 108. The surgeon then applies gentle pressure to the body to ensure the adhesive 118 engages the appropriate portions of the target vessel 104. The application of pressure to engage the adhesive 118 can be alternatively performed prior to the release of the radial compression of the cylindrical body 121.

The surgeon then releases the ligation of the target vessel 104, checks the bypass grafts for adequate blood flow in a conventional manner, and completes the remainder of the operation in a conventional manner.

While there has been shown and described what is considered to be preferred embodiments of the invention, it will, of course, be understood that various modifications and changes in form or detail could readily be made without departing from the spirit of the invention. It is therefore intended that the invention be not limited to the exact forms described and illustrated, but should be constructed to cover all modifications that may fall within the scope of the appended claims.

## Claims

1. A device for creating an end-to-side anastomosis between first and second vessels, the device comprising:
a body, at least a portion of which is fabricated from a sponge material, the body having an opening for insertion of an end of the first vessel therein;
first securing means for securing the first vessel in the opening; and
second securing means for securing a side of the second vessel to the body such that a hole formed in the side of the second vessel is in fluid communication with the end of the first vessel.

2. The device of claim 1, wherein the body is disk-shaped.

3. The device of claim 1, wherein the body is sleeve-shaped.

4. The device of claim 1, wherein the first securing means comprises an adhesive disposed on between an outer surface of the first vessel and a corresponding surface of the opening.

5. The device of claim 4, wherein the first securing means further comprises sealing means for sealing the outer surface of the first vessel against the corresponding surface of the opening.

6. The device of claim 5, wherein the sealing means comprises a catheter having a balloon, the catheter being disposed in the first vessel such that when inflated, the balloon urges the outer surface of the first vessel against the corresponding surface of the opening to sandwich the adhesive therebetween.

7. The device of claim 1, wherein the second securing means comprises an adhesive disposed between an outer surface of the second vessel and a corresponding surface of the body.

8. The device of claim 7, wherein the adhesive is disposed on at least a first vessel-contacting surface of the opening.

9. The device of claim 8, wherein the sponge material contains pores and the adhesive is further disposed in the pores of the sponge material.

10. The device of claim 1, further comprising an alignment means for aligning the opening with the hole in the second vessel.

11. The device of claim 10, wherein the alignment means comprises a radially compressible member having a cylindrical body embedded in the body circumferentially about the opening, the radially compressible member further having a plurality of pins protruding from the body.

12. The device of claim 11, wherein at least a portion of the radially compressible member is fabricated from a resorbable material.

13. The device of claim 1, wherein the sponge material is resorbable.

14. The device of claim 1, wherein the sponge material contains pores and the device further comprising a medicating agent disposed in at least a portion of the pores of the sponge material

15. The device of claim 14, wherein the medicating agent is an anastomosis modulating agent.

16. A device for creating a side-to-side anastomosis between first and second vessels, the device comprising:
a body, at least a portion of which is fabricated from a sponge material, the body having an opening formed therein;
first securing means for securing a side of the first vessel to the body; and
second securing means for securing a side of the second vessel to the body such that a hole formed in the side of the first and second vessels are each in fluid communication with the opening.

17. The device of claim 16, wherein the body is disk-shaped and having a slot of semicircular cross-section corresponding to each of the first and second vessels for acceptance thereof, each of the slots communicating through the opening.

18. The device of claim 16, wherein at least one of the first and second securing means comprises an adhesive disposed between an outer surface of the first and/or second vessel and a corresponding surface of the body.

19. The device of claim 18, wherein the at least one of the first and second securing means further comprises sealing means for sealing the outer surface of the first and/or second vessel against the corresponding surface of the body.

20. The device of claim 19, wherein the sealing means comprises a catheter having a balloon, the catheter being disposed in the first and/or second vessel such that when inflated, the balloon urges the outer surface of the first and/or second vessel against the corresponding surface of the body to sandwich the adhesive therebetween.

21. The device of claim 18, wherein the sponge material contains pores and the adhesive is disposed in pores of the sponge material.

22. The device of claim 16, further comprising an alignment means for aligning the opening with at least one of the holes in the first and second vessels.

23. The device of claim 22, wherein the alignment means comprises a radially compressible member having a cylindrical body embedded in the body circumferentially about the opening, the radially compressible member further having a plurality of pins protruding from the body.

24. The device of claim 23, wherein at least a portion of the radially compressible member is fabricated from a resorbable material.

25. The device of claim 17, wherein the sponge material is resorbable.

26. The device of claim 17, wherein the sponge material contains pores and the device further comprising a medicating agent disposed in at least a portion of the pores of the sponge material.

27. The device of claim 26, wherein the medicating agent is an anastomosis modulating agent.

28. The device of claim 18, wherein at least one of the first and second securing means comprises an adhesive disposed on a surface of the slot for adhering the first and/or second vessel to the corresponding slot.
